# EUROPEAN PATENT SPECIFICATION

(11) **EP 2 983 579 B1**
(45) Date of publication and mention of the grant of the patent: **09.07.2025**
(21) Application number: 14731417.3
(22) Date of filing: 10.04.2014
(51) Int. Cl.: A61B 5/00, G01N 21/17

(54) **MULTIPLE APERTURE, MULTIPLE MODAL OPTICAL SYSTEMS AND METHODS**
OPTISCHE SYSTEME UND VERFAHREN MIT MEHREREN ÖFFNUNGEN UND MEHREREN MODI
SYSTÈMES OPTIQUES À MODES MULTIPLES, OUVERTURES MULTIPLES ET PROCÉDÉS

(30) Priority: 12.04.2013 US 201361811193 P
(43) Date of publication of application: 17.02.2016
(73) Proprietor: NinePoint Medical, Inc., Bedford, MA 01730 (US)
(72) Inventor: NAMATI, Eman, Arlington, MA 02476 (US); SICLAIR, Matthew, A., Stoneham, MA 02180 (US); VADER, David, Brookline, MA 02445 (US); GRAF, Ben, Charlestown, MA 02129 (US)
(74) Representative: WP Thompson
(86) International application number: PCT/US2014/033624
(87) International publication number: WO 2014/169103

(56) References cited:
- WO-A1-2010/064516
- WO-A1-2010/067813
- JP-A- 2001 070 228
- JP-A- 2008 125 939
- JP-A- 2010 210 501
- JP-A- 2010 210 501
- US-A1- 2003 194 050
- US-A1- 2006 146 339
- US-A1- 2007 078 305
- US-A1- 2007 159 639
- US-A1- 2007 282 403
- US-A1- 2008 117 424
- US-A1- 2008 140 328
- US-A1- 2009 131 800
- US-A1- 2012 101 374
- US-A1- 2013 058 553
- US-A1- 2013 178 735
- US-B1- 8 953 911

## Description

### RELATED APPLICATIONS

This application claims priority to U.S. provisional patent application serial number 61/811,193, filed April 12, 2013, entitled MULTIPLE APERTURE OPTICAL SYSTEM.

### TECHNICAL FIELD

The present disclosure generally relates to medical devices, systems and methods for imaging in biomedical and other medical and non-medical applications, and more particularly, to probes, systems and methods for generating an image in a multiple aperture, multiple modal optical system for Optical Coherence Tomography (OCT) imaging.

### BACKGROUND

Various forms of imaging systems are used in healthcare to produce images of a patient. Often, an image of an internal cavity of a patient is required. These cavities can include areas of the digestive system and/or the respiratory system. Surgical incisions are also used to access internal cavities. When imaging tissue features of these systems, fiber optic endoscopy is often utilized.

One type of fiber optic endoscope is based on Optical Coherence Tomography (OCT) techniques. OCT provides structural information on tissue with high resolution. OCT can provide this information in real time and in a non-invasive manner. One example is disclosed in U.S. patent application serial number 13/365,621, filed February 3, 2012, entitled IMAGING SYSTEM PRODUCING MULTIPLE REGISTERED IMAGES OF A BODY LUMEN.

Many different lens types have been used to construct fiber optic endoscopes. These lenses include fiber lenses, ball lenses and GRadient INdex (GRIN) lenses. Lens materials can vary from glass to plastic to silicon. An optical probe must be specifically manufactured to conform to optical parameters required for a specific use.

In addition, many different energy types with proper probes are used in fiber optic endoscopy. For example, coherent laser light can be used for deep tissue scans, visible light for surface imaging, and ultrasound for intravascular imaging.

Light or energy from a source is focused onto or into the tissue. The tissue scatters the light or energy and the light or energy that is reflected back to the probe is received at a detector that converts the light to electrical signals. A processing system is used to analyze the detected light (i.e. the electrical signals) and produce images on a display. These images can be manipulated to produce variations for better diagnosis by a health care professional.

Esophageal imaging requires probes of specific design to properly image into surrounding tissue. Typical esophageal imaging systems include a prism to direct light off axis into the surrounding tissue. In order to produce a full image of an esophagus the probe must be rotated within the esophagus at a specific rotation rate and translated along the esophagus at a specific translation rate throughout the scanning process. If the rotation rate and/or the translation rate are too fast for a proper scanning, the image produced will be rendered useless. Whereas a slower rotation and/or translation rate increases the costs of imaging.

The typical optical imaging system consists of a single optical probe and a single energy (e.g. visible light) source. A particular optical probe has a set characteristics used for specific image requirements. Such characteristics can include, for example, depth of field, polarization, resolution, visible imaging, etc. Thus, if multiple characteristics are required, multiple scans using multiple probes must be performed.

If a multiple scan is performed, the multiple images must often be viewed individually due to scaling and alignment problems. If two images are to be viewed together as one composite image, they will be distorted and useless unless properly scaled and aligned.
Documents US2008/117424A1, US2012/101374A1, US2003/194050A1, US2007/078305A1 and US2007/282403 A1 disclose a multiple modal optical system according to prior art.

This disclosure describes improvements over these prior art technologies.

### SUMMARY

Accordingly, a multiple modal optical system according to claim 1 is provided.

Accordingly, a multiple modal optical method according to claim 6 is also provided.

### BRIEF DESCRIPTION OF THE DRAWINGS

The present disclosure will become more readily apparent from the specific description accompanied by the following drawings, in which:
FIG. 1 is a diagram illustrating an Optical Coherence Tomography (OCT) optical probe system;
FIG. 2 is a cross sectional diagram illustrating the system of FIG. 1 along line A-A;
FIG. 3 is a diagram illustrating various operating parameters of an optical probe;
FIG. 4A is a diagram illustrating a multiple aperture optical probe system according to the present disclosure;
FIG. 4B is a diagram illustrating a single aperture, multiple modal optical probe system according to the present disclosure;
FIG. 5 is a cross sectional diagram illustrating the system of FIG. 4 along line A-A according to the present disclosure;
FIG. 6 is a graph illustrating Acoustic Optical Frequency (AOF) shifting according to the present disclosure;
FIG. 7 is a diagram illustrating the use of multiple path mirrors in the light path according to the present disclosure;
FIG. 8 s a graph illustrating path length shifting according to the present disclosure;
FIG. 9 is a diagram illustrating a first configuration of a multiple aperture optical system.
FIG. 10 is a diagram illustrating a second configuration of a multiple aperture optical system
FIG. 11 is a diagram illustrating a third configuration of a multiple aperture optical system
FIG. 12 is a diagram illustrating a fourth configuration of a multiple aperture optical system
FIG. 13 is a diagram illustrating a fifth configuration of a multiple aperture optical system.
FIG. 14 is a diagram illustrating a sixth configuration of a multiple aperture optical system
FIG. 14A is a diagram illustrating an imaging fiber having multiple cores;
FIG. 15 is a flow chart illustrating the method for generating an image in a multiple aperture optical system
FIGs. 16A, 16B, 17A, 17B and 18 are diagrams illustrating a first example according to the present disclosure;
FIGs. 19A, 19B, 20A, 20B and 21 are diagrams illustrating a second example according to the present disclosure;
FIGs. 22A, 22B, 23, 24A and 24B are diagrams illustrating a third example according to the present disclosure;
FIGs. 25A, 25B, 26A, 26B, 27A, 27B, 27C are diagrams illustrating a fourth example according to the present disclosure;
FIGs. 28-30 are diagrams illustrating a correlation process;
FIG. 31 is a diagram illustrating non-uniform rotational distortion;
FIG. 32 is a diagram where the non-uniform rotational distortion is at a minimum;
FIG. 33 is a diagram of hardware components in a multimodal OCT system according to the present disclosure;
FIG. 34 is a diagram illustrating white light or narrowband imaging having discrete wavelengths in a multimodal OCT system according to the present disclosure;
FIG. 35 is a diagram illustrating white light, narrowband or hyper spectral imaging having continuous wavelength band in a multimodal OCT system according to the present disclosure;
FIG. 36 is a diagram illustrating fluorescence imaging in a multimodal OCT system according to the present disclosure;
FIG. 37 is a diagram illustrating fluorescence imaging with lock in amplifier detection in a multimodal OCT system according to the present disclosure; and
FIG. 38 is a diagram illustrating the generation of white light and narrowband images from raw multimodal/multi-aperture in a multimodal OCT system according to the present disclosure data.

Like reference numerals indicate similar parts throughout the figures.

### DETAILED DESCRIPTION

The present disclosure may be understood more readily by reference to the following detailed description of the disclosure taken in connection with the accompanying drawing figures, which form a part of this disclosure. It is to be understood that this invention is defined by the appended claims.

Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure.

A multi-aperture probe has the significant advantage of co-registering all the different types of data on top of each other compared to a single aperture probe. Additional data is only as good as how well it is located with the OCT image. Characteristics of a multi-aperture probe include faster imaging, higher resolution radially and axially, increased depth of imaging, increased field of view, handle higher optical power (multimode fiber, GRIN fiber, and double clad fiber), white light imaging, and structural information based on polarization.

Multiple aperture, multiple modal optical systems and methods according to the present disclosure can increase the scanning area and/or speed without increasing the rotation rate or decreasing the pitch. The pitch of a pull-back is described the same way as the pitch on the threads of a screw. If the pitch is kept constant, then the resolution will be about double. If the pitch becomes twice as large, then imaging will take half the time and resolution will be maintained assuming 2 identical OCT apertures. Inversely, decreasing the pitch increases the scan time if rotation rate is maintained. Additionally, multiple aperture optical systems and methods according to the present disclosure can extend the depth of field for imaging while maintaining resolution by providing multiple apertures having separate working distances. Still further, multiple aperture optical systems and methods according to the present disclosure can significantly increase resolution by changing the direction of the spectral signal. In addition, multiple aperture optical systems and methods according to the present disclosure can gain additional polarization data by having multiple polarization states. Still yet further, multiple aperture optical systems and methods according to the present disclosure can include a visible imaging modality similar to a regular camera as one of the probe characteristics. Also, multiple aperture optical systems and methods according to the present disclosure can utilize a multimode fiber for a high power laser with a separate OCT channel.

Although many of the embodiments disclosed herein relate to systems having 2 or more distinct apertures, a single aperture configuration is contemplated. The single aperture configuration uses the single aperture to direct and focus at least 2 different energy (e.g. white light and coherent light) sources onto and/or into the surrounding tissue of interest and receive the reflected light back through the aperture. Once received, the processing of the signals is similar to the multiple aperture systems.

The multiple aperture optical systems and methods according to the present disclosure can be realized using a computer that receives signals from two or more optical probes assembled into one functional probe that utilizes multiple path lengths, carrier frequencies, types of fiber, polarizations and/or detectors to separate the image data from each optical probe. A computer receives signals representing images from the optical probe and scales and/or aligns the images to produce a composite image for display.

Reference will now be made in detail to the exemplary embodiments of the present disclosure, which are illustrated in the accompanying figures.

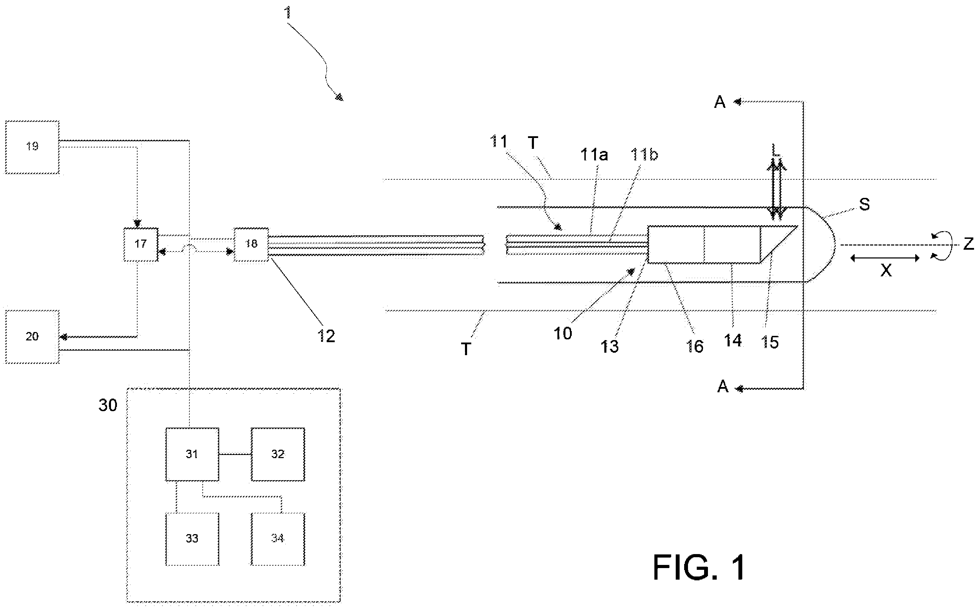

As shown in FIG. 1, an imaging system 1 using an OCT probe 10 for a fiber optic endoscope is comprised of an optical fiber 11 having a casing 11a, a fiber core 11b, a proximal end 12 and a distal end 13, a spacer 16 connected to the distal end of the optical fiber 11, a GRIN lens 14 connected to spacer 16, and a prism 15 connected to GRIN lens 14 and configured to deflect light into surrounding tissue T. Spacer 16 is positioned before the GRIN lens to modify the optical parameters. The fiber core 11b, GRIN lens 14, prism 15, and spacer 16 are typically connected by fusing the components together or using an optical-grade epoxy to glue the components together.

A GRIN lens is described herein for illustrative purposes. Other lenses and lens structures are contemplated. For example, ball lenses, fiber optic lenses, and molded lenses (all of these may be made with or without a grating) can be utilized as the probe without departing from the scope of the present invention.

Probe 10 is typically contained within a sheath S. Sheath S containing probe 10 is inserted into a cavity of a patient to image into tissue T surrounding probe 10. Sheath S protects probe 10 and tissue T from damage.

Probe 10 is typically connected to a light source 19 at proximal end 12 of optical fiber 11 through a rotary junction 18 and optical components 17. Also included is a detector 20 to detect light reflected back from tissue T. The optical components 17 can include elements to direct light from light source 19 toward probe 10 and elements to direct light from probe 10 to detector 20. The energy from light source 19 can be, for example, coherent, visible, infrared (IR) or ultrasound; other energy sources are contemplated.

System 1 is shown connected to computer 30. Computer 30 can include a central processing unit (CPU) 31 for controlling the overall operation of the system, a memory 32 for storing programs upon which the system can operate and data, an input device 33 for receiving input commands from a user, and a display 34 for displaying processed or raw data for images or other information. Computer 30 provides control for the components of system 1. Computer 30 also provides image processing functions to produce images from light detected at detector 20. Input device 33 can include a keyboard and/or a mouse. Output device 34 can include a display for displaying, for example, instructions and/or images.

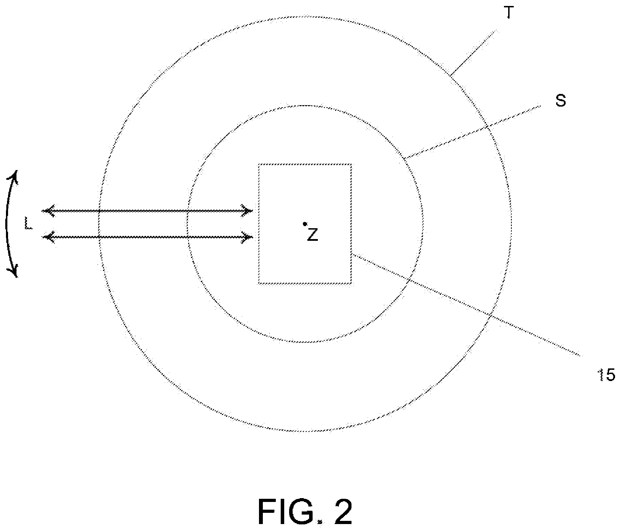

In operation, and also with reference to FIG. 2, light L travels from light source 19, through optical components 17, rotary junction 18, optical fiber 11, spacer 16, lens 14 and prism 15 and into tissue T. Light L is reflected back from tissue T, through prism 15, lens 14, spacer 16 and optical fiber 11, and is directed by optical components 17 to detector 20.

In order to provide an image of a particular area of tissue T, probe 10 is translated along direction X and rotated about axis Z. The translation rate and rotation rate must be maintained at a predetermined and/or known rate to ensure a complete and accurate scan is performed. Anomalies can result if the translation and/or rotation rates are too high, too low or varies over time, a phenomenon referred to as Non-Uniform Rotational Distortion (NURD).

This translation and rotation directs light L into tissue T at an area of concern. In order to produce a complete radial scan of tissue T surrounding probe 10, probe 10 must be rotated 360 degrees to produce an image of a first slice of tissue T and then translated along direction X to produce an image of an adjacent slice of tissue T. This rotation/translation process continues along direction X until the area of concern of tissue T is completely scanned.

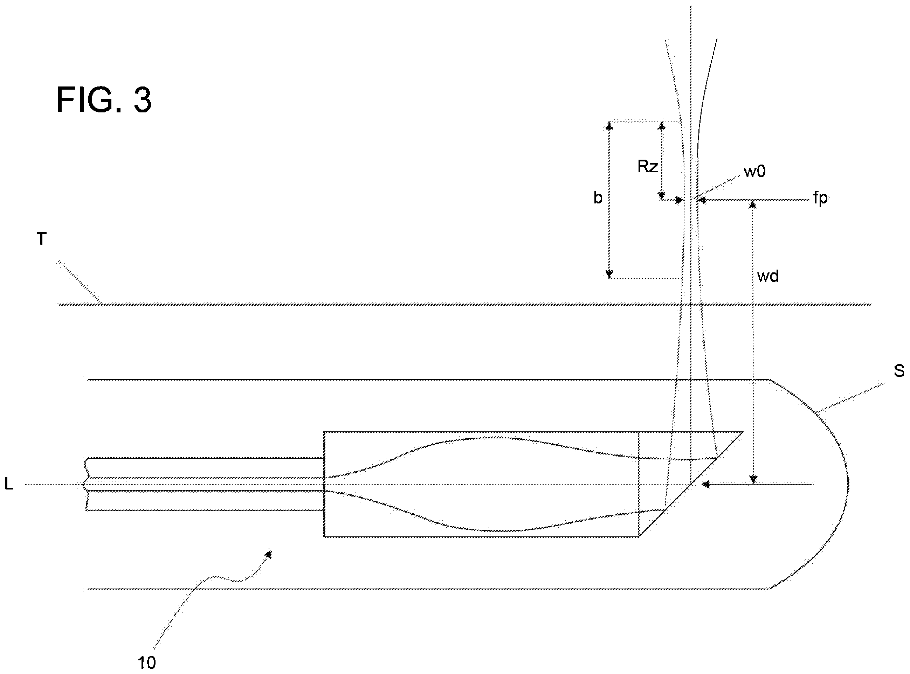

Referring to FIG. 3, proper imaging into tissue using an OCT probe required strict compliance to probe specifications in order to precisely set the optical parameters. These parameters can include the Rayleigh Range Rz, the confocal parameter b, the waist w0, the focal point fp, and the working distance wd. The term "beam waist" or "waist" as used herein refers to a location along a beam where the beam radius is a local minimum and where the wavefront of the beam is planar over a substantial length (i.e., a confocal length). The term "working distance" as used herein means the distance between the optical axis aligned with the fiber and the focal point fp.

An optical probe must be specifically manufactured to conform to required optical parameters. Esophageal imaging requires probes of specific design to properly image into surrounding tissue T. When using an optical probe for esophageal imaging, a long working distance with large confocal parameter is required. Generally in esophageal imaging the working distances from the center of the optical probe radially outward to the tissue ranges from 6 mm to 12.5 mm. The optic itself can be 1 mm in diameter, with a protective cover (not shown) in sheath S and with a balloon (not shown) on top, while still fitting through a 2.8 mm channel in an endoscope. With no tight turns required during the imaging of the esophagus (compared, for example, to the biliary system, digestive system or circulatory system), an optical probe can be as long as 13 mm in length without a interfering with surrounding tissue T. In attempts to manufacture an optical probe that conforms to these parameters, several designs have been utilized. Ball lenses, GRadient INdex (GRIN) lenses, and molded lenses may be used with or without an outer balloon structure can increase the working distance and achieve better imaging conditions.

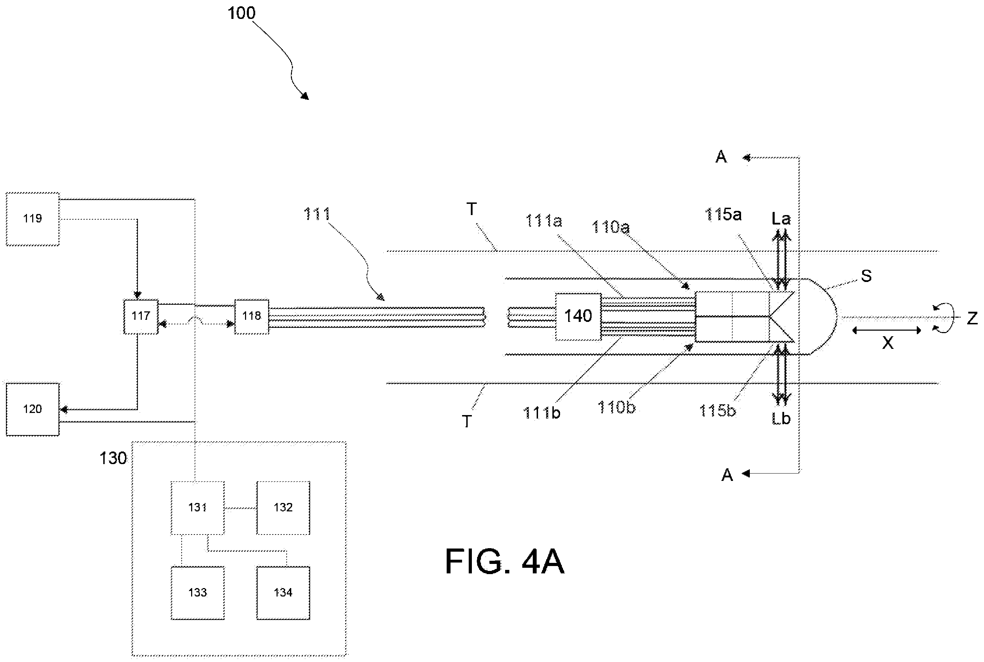

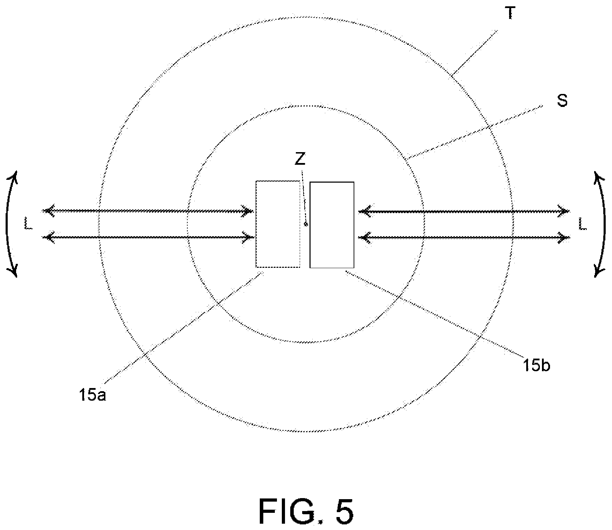

A multiple aperture optical system in accordance with one embodiment of the present disclosure is illustrated in FIGs. 4A and 5. The multiple aperture optical system 100 includes probes 110a and 110b combined to form a single probe assembly. Each probe 110a/110b has a distinct aperture, illustrated as prism 115a and prism 115b. As stated earlier, other probe structures can be used to produce the multiple aperture structure. Prisms 115a/115b are positioned to direct and receive light La and Lb in directions different from each other. As shown in FIGs. 4A and 5, light La and Lb are directed 180 degrees from each other and within the same trans-axial plane. Although shown with only 2 probes, other configurations with more than 2 probes are also contemplated, for example, a configuration having 3 probes that offset light at 120 degrees from each other; other offsets are contemplated and it is not required that the offsets be equidistant. The number of probes that can be incorporated is only limited by the space available in the area being imaged and the size of the probes themselves. Also, although light La and Lb are shown within the same trans-axial plane offsets are contemplated.

Each probe 110a/110b is connected to an optical fiber 111a and 111b that connects a respective probe 110a/110b to at least one light source 119. A concept of the present disclosure is to provide each probe 110a/110b with light that when received back at detector 120 can be used during the image processing.

In one embodiment, each probe 110a/110b is connected to a distinct coherent light source that requires a multiple channel rotary junction 118 to handle the multiple light paths.

In another embodiment a single light source 119 can be utilized while employing an optical component 140 that functions to split light path through optical fiber 111 from light source 119 into 2 separate light paths, each having a fraction of the total power and each having a different path length. In this embodiment only a single channel rotary junction 118 would be required. As stated above, light source 119 can be any available energy source for different imaging requirements or imaging modes, e.g. coherent light or ultrasound.

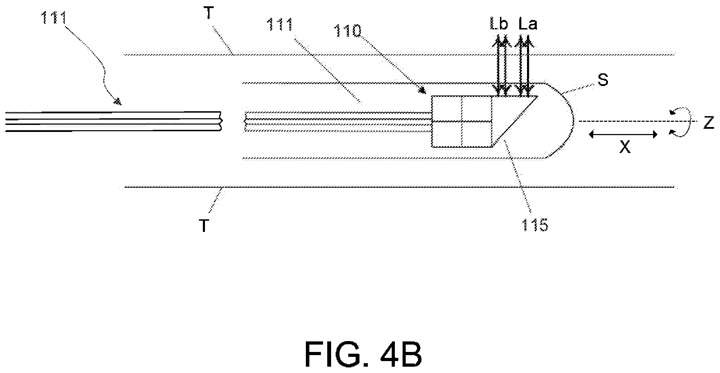

A single aperture multimodal OCT optical system in accordance with the present disclosure is illustrated in FIG. 4B, using a single aperture configured with special characteristics capable of supporting multiple modalities. A photonic crystal fiber can be used to support a single mode operation from visible to near-infrared. A photonic crystal fiber supports multiple wavelengths using an air core with internal air holes.

The single aperture optical system is similar to the multiple aperture system of FIG. 4A which includes probe 110. The probe 110 uses a single aperture, illustrated as prism 115. The single fiber and single aperture is capable of carrying multiple modalities of light/energy. Prism 115 is positioned to direct and receive both light La and Lb in the same direction. As shown in FIG. 4B, light La and Lb are directed in the same direction. In either the single or multiple aperture configuration, the concepts described herein are applicable.

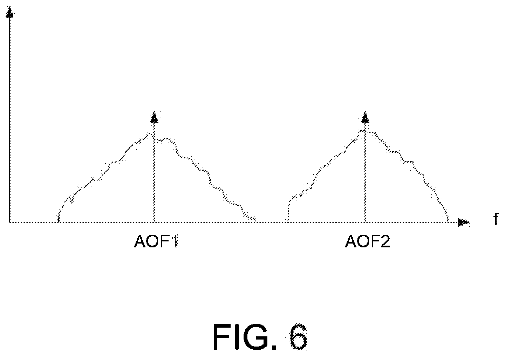

In yet another embodiment the light can be multiplexed to the multiple probes using Acoustic Optical Frequency (AOF) shifting. As shown in FIG. 6, the frequency of probe 110a is positioned at AOF1 and the frequency of probe 110b is positioned at AOF2. This permits sufficient separation of the detected light to perform accurate image processing.

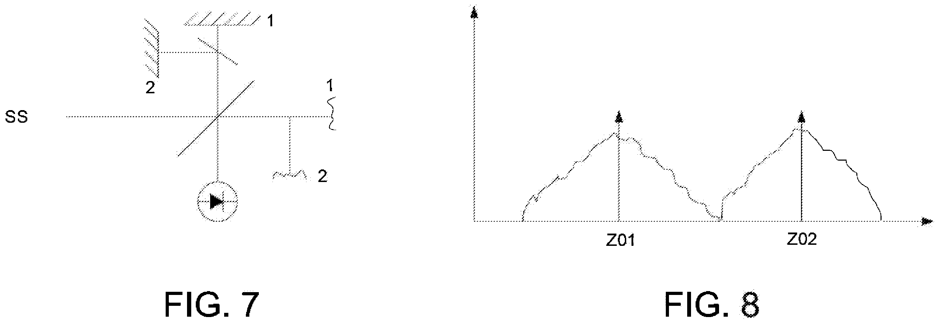

In still yet another embodiment the light paths of differing path lengths can be used. As shown in FIGs. 7 and 8, multiple path lengths can be created using mirrors and probes of differing path lengths. The zero path length point of probe 110a is positioned at Z01 and the zero path length point of probe 110b is positioned at Z02. This again permits sufficient separation of the detected light to perform accurate image processing. Shown in FIG. 7 are reference arm and mirror path length 1 for Z01 and reference arm and mirror path length 2 for Z02. In FIG. 8, on the left is illustrated the sample that produces an interference pattern associated with reference mirror Z01 and on the right is illustrated the sample that produces an interference pattern associated with reference mirror Z02.

In still yet another embodiment the light travels down one or more stationary optical fibers to the distal end of the probe where a mechanism is employed to scan the tissue of interest. The scanning mechanism can result in moving the light beam in a linear pattern (X, Y or Z), raster pattern (X-Y, Z-Y), rotational pattern (theta) or combination thereof. Systems and/or methods of scanning could include MEMS mirror, linear or rotary motors, piezo elements or combinations thereof.

Other methods are contemplated. Whichever method is utilized, an object of the present disclosure is to provide light to the probe(s) to generate at least 2 reflections and to be able to distinguish between the reflected light for further image processing occurring in computer 130.

Additionally, the present disclosure is described as using a coherent light source as the at least one light source 119, but additional configurations are possible. Other configurations include a visible light source to provide a visual imaging mode or an ultrasound energy source to produce an ultrasound image. Several of these configurations will be discussed in further detail below.

Although a GRIN lens is described herein for illustrative purposes, other lenses and lens structures are contemplated. For example, ball lenses, fiber optic lenses, molded lenses, and molded multi-aperture probes (all of these may be made with or without a grating) can be utilized as the probe without departing from the scope of the present invention.

Probes 110a/110b are typically contained within a sheath S, which is insertable into a cavity of a patient to image into tissue T surrounding probes 110a/110b to protect probes 110a/110b and tissue T from irritation or damage.

Probes 110a/110b are connected to a coherent light source 119 through optical fiber 111, rotary junction 118 and optical components 117. Also included is detector 120 to detect light reflected back from tissue T. The optical components 117 can include elements to direct light from light source 119 toward probes 110a/110b and elements to direct light from probes 110a/110b to detector 120.

System 100 is shown connected to computer 130. Computer 130 provides control for the components of system 100. Computer 130 also provides image processing functions to produce images from light detected at detector 120. Computer can include CPU 131 and memory 132. Computer 130 can also include one or more input devices 133 such as a keyboard and/or a mouse. Computer 130 can also include one or more output devices 134 such as a display for displaying, for example, instructions and/or images.

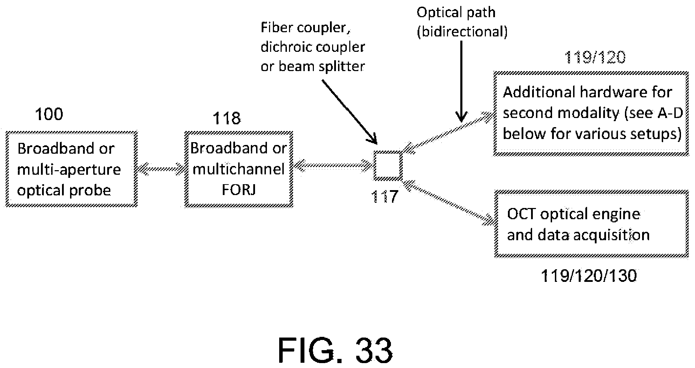

FIG. 33 illustrates an overview of the imaging options of the multiple aperture-multimodal OCT system. Various imaging options are available in the present invention. Although not inclusive, hardware setups for multiple modal and/or multiple aperture imaging options can include integrated OCT and point scanning visible/narrowband imaging, fluorescence imaging (either auto fluorescence or from a fluorescent dye that has been applied to the tissue surface), blood vessel imaging (Doppler or phase variance OCT imaging), dual band OCT (for example two separate OCT systems, one centered at 800 nm and the other at 1300 nm), confocal microscope, and/or spectrally encoded confocal microscopy.

With respect to the OCT imaging, the following energy/light sources are contemplated: Red-Green-Blue (RGB) laser diodes and combiner to provide the three channels needed to reproduce visible imaging and narrow-band imaging, and/or broad bandwidth source (such as a super continuum laser) for continuous visible imaging or hyper spectral imaging. Other sources are also contemplated. The detectors can include several individual detectors for different laser wavelengths and/or a spectrometer. The detection schemes that can be utilized can consist of a direct scheme (simply measure the intensity of backscattered light) and/or a lock in amplifier based theme (sinusoidaly modulate the light source and use a lock in amplifier in the detector electronics to boost sensitivity).

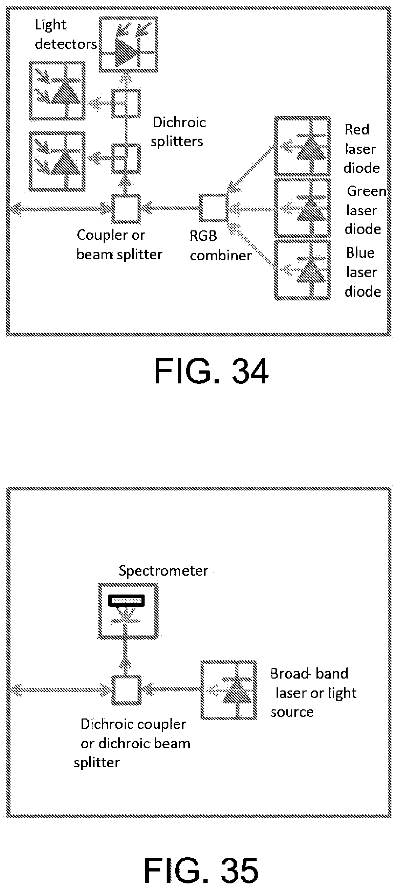

Turning again to FIG. 33, a general overview of the components of the major subsystems of a multimodal/multiple aperture OCT imaging system will now be described. In addition to the OCT optical engine and data acquisition system (see, e.g. FIG. 4A at 119/120/130) there is an additional hardware setup for the second imaging modality (see, e.g. FIG. 4A at 119/120 and FIGs. 34-37). The optical outputs from these two modules can be combined using a Wavelength Division Multiplexor (WDM) or coupler. Alternatively the two optical paths can be connected to the appropriate channel of the multi aperture probe in a multichannel Fiber Optic Rotary Junction (FORJ).

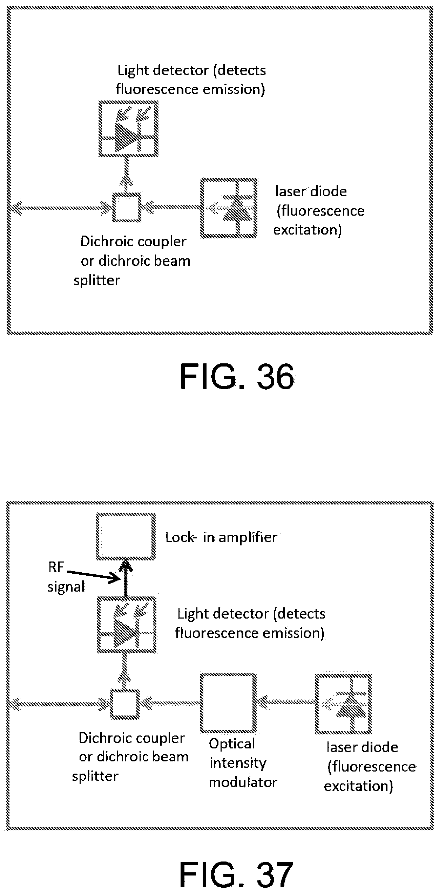

FIGs. 34-37 are diagrams illustrating various hardware configurations for modalities/multiple apertures other than OCT imaging. FIG. 34 is a configuration for utilizing white light and/or narrowband imaging (discrete wavelengths) using separate laser sources and detectors for generating a red, green and blue color channel. The narrowband imaging can be performed by the narrowing of the visible light spectrum used to image hemoglobin using the green-blue or blue-green wavelengths. FIG. 35 illustrates a configuration for a single light source with a broad spectral bandwidth and a spectrometer as the detector. This white light, narrow band or hyper spectral imaging is a continuous wavelength band. FIG. 36 illustrates a configuration required to perform fluorescence imaging and FIG. 37 illustrates a configuration for an alternative fluorescence imaging setup which uses modulated light and a lock in amplifier to boost detection sensitivity to lock into a particular modulation frequency. The preceding configurations are exemplary in nature and not meant to be an exclusive listing of configurations.

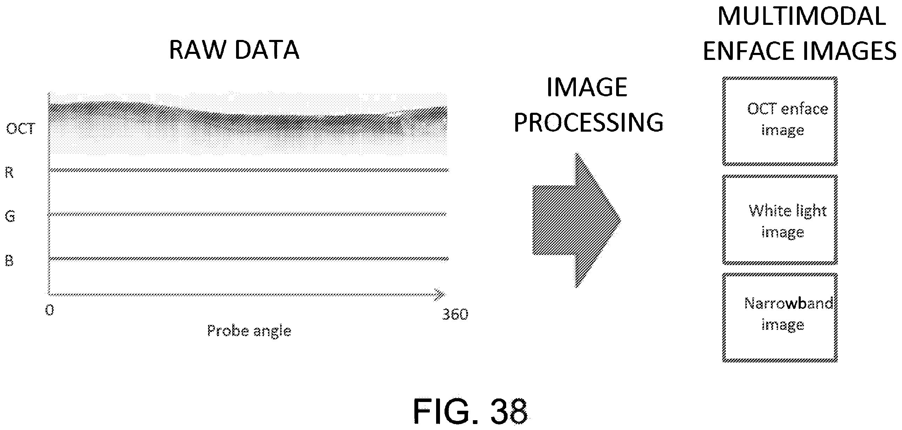

FIG. 38 illustrates the generation of multi-modal images from the raw data that is collected from a multiple aperture and/or multiple modal OCT system. FIG. 38 illustrates a white light and narrowband imaging example. The raw data for a single rotation of the probe consists of a 2 dimensional (2D) OCT image along with 1 dimensional (1D) datasets for each of the red, green and blue intensity channels. The raw data from many rotations can be combined using image processing to generate a final set of enface images. The OCT enface image is generated by transforming each raw 2D OCT image to a single line of data by projecting along the axial dimension. Each line is then represented as one line in the final gray scale enface image. The RGB lines from each rotation can be combined on an RGB color scale to form a single line in the final white light or narrowband image. The difference between the white light image and the narrowband image is the particular weighting of each of the different color channels.

In operation, and also with reference to FIG. 5, light L travels from light source 119, through optical components 117, rotary junction 118, optical fiber 111, optical component 140 (where applicable) where it is split into 2 paths, into fibers 111a/111b, through probes 110a/110b and into tissue T. Light La and Lb is reflected back from tissue T, through probes 110a/110b, optical fibers 111a/111b, optical component 140 where it is merged into single optical fiber 111, and is directed by optical components 117 to detector 120. Detector 120 converts the detected light into electrical signals that are forwarded to computer 130 for processing, which includes scaling and/or alignment of the images produced from the multiple signals.

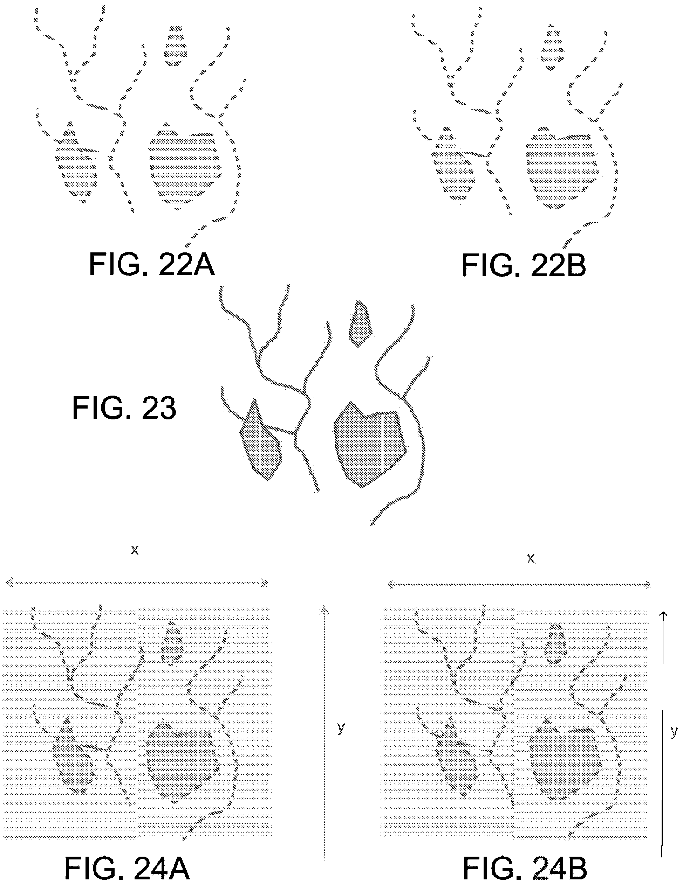

In order to provide an image of a particular area of tissue T, probes 110a/110b undergo a similar translation along direction X and rotation about axis Z. The translation rate and rotation rate must be maintained at a predetermined rate to ensure a complete and accurate scan is performed. In the preset disclosure, if the same type of probe is used for both probe 110a and probe 110b, the rotation rate can be maintained while doubling pitch between translations as the image is 1/2 obtained by probe 110a and 1/2 obtained by probe 110b and then combined by computer 120 to form a complete rotational scan. A faster acquisition can be obtained thus saving cost and time of the imaging process. Computer 130 can utilize a type of interleaving process when combining images from the same types of probes, i.e. the same imaging modes. The interleaving process is also discussed below with respect to FIGs. 22-24.

As discussed above, multiple probes are utilized to provide multiple images from a single pass of an OCT imaging system according to the present disclosure. Different configurations of types of probes can be combined to produce varying imaging results. One configuration where two similar probes are used to reduce the acquisition time was described above. The following are illustrations of various configurations according to the present disclosure; other configurations are contemplated.

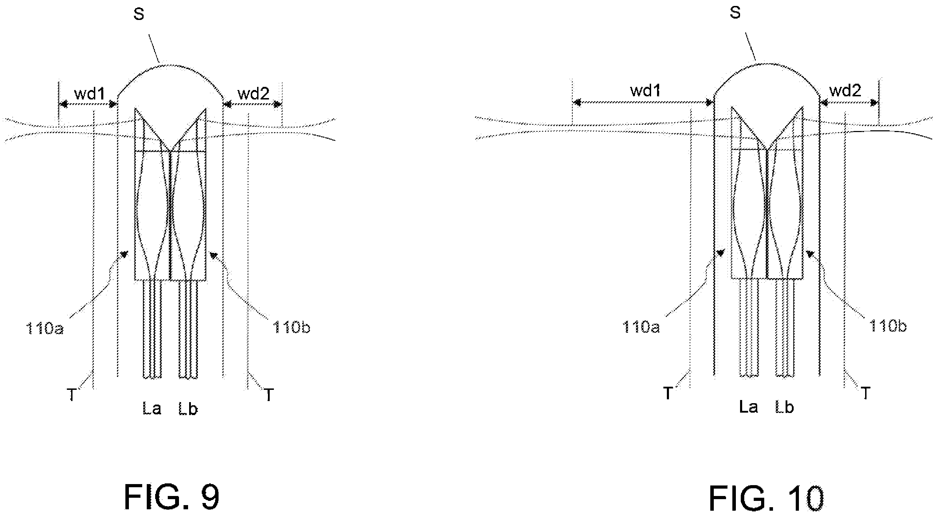

FIG. 9 illustrates a configuration comprised of 2 probes 110a/110b having approximately the same prescription. The working distances wd1 and wd2 would be approximately the same. Resolution would be maintained while producing faster imaging since 2 similar probes can image the same area in half the time. Light La and Lb can be transmitted through separate OCT channels.

FIG. 10 illustrates a configuration comprised of 2 probes 110a/110b having different prescriptions. The working distances wd1 and wd2 are different, that is, one probe 110a will image deeper into tissue T that the other probe 110b. This configuration can produce an image or images having different depths without the need to conduct 2 separate imaging processes as in the present state of OCT imaging. Light La and Lb can be transmitted through separate OCT channels or the same channel if they are separate wavelengths or polarizations.

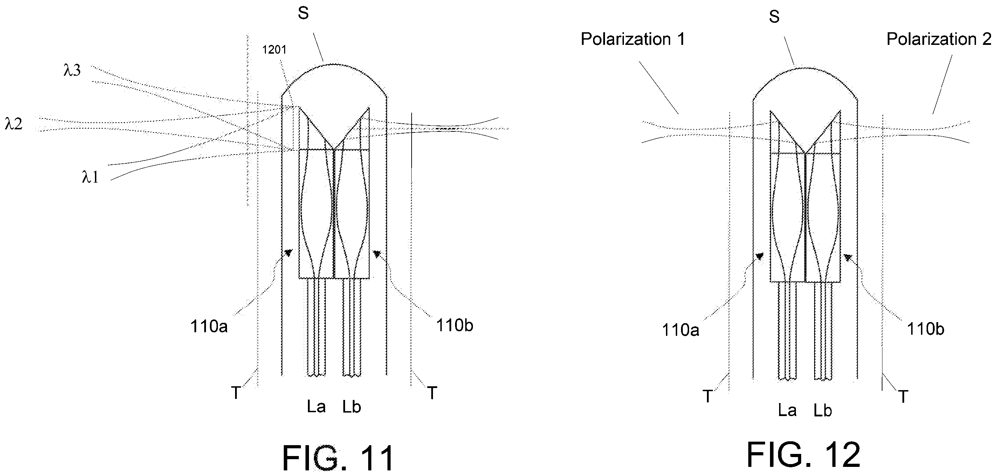

FIG. 11 illustrates a configuration comprised of 2 probes 110a/110b having different points of resolution thus extending the depth of field. In this configuration, probe 110a includes a spectral grating 1201 to diffract light into separate wavelengths (λ). The dotted lines show the spectrally encoded resolutions. The light from probe 110a should be focused at 1 Rayleigh range into the tissue, though variations are contemplated. As shown, probe 110a has a high resolution radially thus capable of producing a high resolution image of the surface of tissue T and probe 110b has a high resolution axially thus capable of producing a high resolution image of some point into tissue T. Light La and Lb can be transmitted through separate OCT channels.

FIG. 12 illustrates a configuration comprised of 2 probes 110a/110b having different polarizations. The polarizations can be S and P or 90 degree out of phase; other polarizations are contemplated. Using the different polarizations, the polarization of tissue T can be extrapolated therefrom rather than requiring a direct determination of the polarization of tissue T in a separate process. Light La and Lb can be transmitted through separate OCT channels.

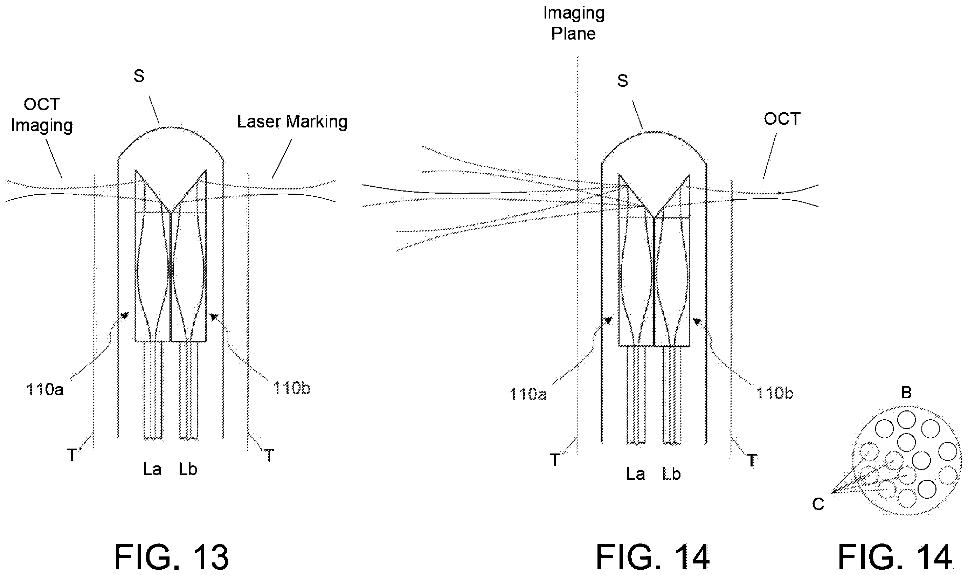

FIG. 13 illustrates a configuration comprised of 2 probes 110a/110b of completely different types. In this configuration illustrated in FIG. 13, probe 110a is an OCT probe and probe 110b is a laser marking probe. The laser marking probe can be used to mark tissue T for later identification in an image for positional reference or removal of tissue. A single mode, multimode, GRIN, or double clad fiber can be used to supply the required light/laser power. That is, the imaging light La can be supplied through a single mode fiber and the laser marking light Lb can be supplied through an inexpensive multimode fiber (e.g. a single mode core with an 85 micron cladding and a 125 micron double cladding); variations are contemplated.

Another embodiment similar to that shown in FIG. 13 can include a configuration where probe 110a is an OCT probe, and probe 110b is a laser therapy or laser treatment probe, e.g. for use in laser surgery. It is also contemplated that three probes, e.g. an OCT probe, a laser marking probe and a laser therapy probe, are all included and can permit OCT imaging, laser marking, and laser treatment all in one device.

FIG. 14 illustrates a configuration comprised of 2 probes 110a/110b again of completely different types. In this configuration illustrated in FIG. 14, probe 110a is an imaging probe and probe 110b is an OCT probe. FIG. 14A illustrates an imaging bundle (e.g. 2000 cores C in one bundle B). The imaging bundle with illumination channels can supply light La; some cores are for transmission and others for reception. An OCT channel can supply light Lb.

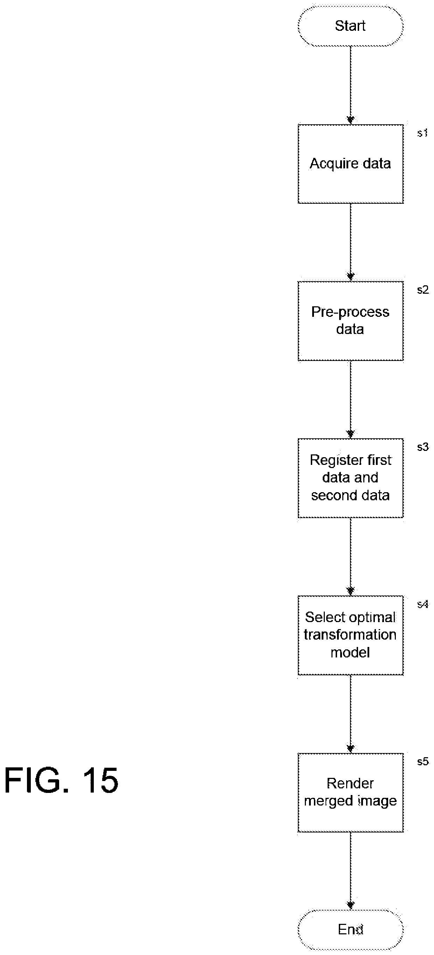

FIG. 15 is a flow chart illustrating the method for generating an image in a multiple aperture, multiple modal optical system according to the present disclosure. Generally, an image produced by the present invention is a 3-dimensional volume of data, where each VOlumetric piXEL (VOXEL/voxel) has an associated intensity. While the three traditional dimensions are x, y and z, it may be, in some cases, more convenient to represent the data in cylindrical coordinates (i.e. r, theta and z), particularly in a rotating probe setup. The image data collected from probe 1 is denoted by im1 and the image data collected from probe 2 is denoted by im2.

In a system having multiple probes (or one probe having multiple modes, e.g. see FIG. 4B), when trying to combine the two (or more) datasets, two situations issues arise. In a first case, where both probes use the same imaging modality, and thus allow faster and/or higher resolution image acquisition, the combining of the two datasets is similar to working with interleaved data in video streams. In a second case, each probe uses a different imaging modality, for example, one of the probes could be processing OCT imaging with infrared and the other one could be collecting white light reflection intensity from the tissue surface, or for example, where one of the imaging modalities is 3-dimensional while the other is 2-dimensional.

In step s1 the data is acquired from the imaging system through the detectors as described above. As many different modes are available, all of the modes are contemplated and the system can easily be adapted to manage the varying datasets.

In step s2 preprocessing is performed. As will be described later, if images are to be produced to represent the raw (pre-processed) data, this step may be eliminated. If subject to preprocessing, the data of im1 and im2 is processed to obtain im11 and im21, respectively. Preprocessing can include processes to clean the images of known image artifacts (e.g. precession, saturation) and normalize them, that is, correct for other artifacts such as decentering and intensity variations. This step may also include, but is not limited to, background subtraction, shifting of data along axes to correct for precession and decentering, masking of saturated lines, and/or normalization by subtraction of a global or local mean and possible division by global or local measure of variability, e.g. standard deviation. In addition, cropping and/or resampling of data to a normalized, convenient size may also be performed.

In step s3 a registration of the 2 data sets is performed. This step can also include alignment and scaling of the data for the 2 images. It is in this step that a geometric transformation model is determined that will be used to map the voxel coordinates of im1 onto those of im2. Registration can include one or more of the following processes, or a combination thereof.

One registration technique is based on statistical methods, e.g. optical flow, Normalized Cross-Correlation (NCC), and Particle Imaging Velocimetry (PIV). These statistical methods for registration attempt to match regions of interest of a given size from one data set with regions of interest of the same size in the other data set. The process is performed by computing the statistical correlation between the pixel values of those two regions of interest or between a transformed set of pixel values of those two regions of interest. Other common registration techniques are contemplated. Other such registration techniques can include, for example, those disclosed in J. P. Lewis, Fast Normalized Cross-Correlation, Vision Interface (1995), pp. 120-123; Horn, Berthold KP, and Brian G. Schunck, Determining optical flow Artificial intelligence 17.1 (1981), pp. 185-203; Adrian, Ronald J. Particle-imaging techniques for experimental fluid mechanics, Annual review of fluid mechanics 23.1 (1991), pp. 261-304.

Other registration techniques are feature-based methods, e.g. using feature detectors and descriptors (e.g. Speed Up Robust Features (SURF), Scale-Invariant Feature Transform (SIFT), Local Binary Patterns, (LBP), Histogram of Gradients (HoG)) and finding the optimal feature pairs in the two datasets that satisfy a given transformation model (e.g. rigid body rotation, translation, scaling, affine transformation, etc...) First, salient feature locations are detected in datasets 1 and 2. These salient features represent voxel locations, in the datasets, where features of interest may be located: typically, blobs of significantly higher or lower intensity. At each of these locations, a feature vector is computed based on one of the vector models (e.g. SURF, SIFT, LBP, HoG) depending on the nature of the features to be determined. Next the feature vector pairs are determined (one from dataset 1 and one from dataset 2) that are nearest neighbors and that most resemble each other in feature space. Finally, based on all the nearest neighbor feature pairs detected between the two datasets, a geometric transformation model is determined that satisfies specific criteria of smoothness and plausibility, while minimizing the number of outliers in the matched pairs.

Additional methods for registration, alignment and/or scaling of the two datasets can include tracking of a known (existing or created) target to calibrate the offset and/or scaling between the multiple datasets. This process can be performed as a calibration step for each probe, or can be done in real time during image acquisition. For example, in the case of a balloon-based probe, a surface or surface marker of the (cylindrical) balloon can be tracked and used to match the two or more image sets based on the detected surface or marker, that is, a type of feature-based method. Tracking of the balloon surface would be much faster than any of the feature descriptors mentioned above. A real-time registration and merge rendering of multiple aperture datasets can be performed.

In step s4 an optimal transformation model is selected. The selection is based, at least in part, on criteria such as number of outliers, closeness of feature positions and descriptors, regularity of the geometric transformation, other a priori knowledge of the expected optical and mechanical geometry linking the two (or more) probes and their associated images. Following statistical and/or feature-based registration, the optimal transformation model is applied to image set im1, or im11 if preprocessing is performed. As discussed above, there is the option of working with either image set im1 or im11 or any other intermediate steps between a raw (i.e. unprocessed) dataset and a full processed dataset. The choice of at which stage of preprocessing the data ends up being used for displaying and rendering, depends on the accuracy and confidence in the preprocessing steps, and/or how close the user wants to remain to the unprocessed (i.e. raw) dataset.

In step s5 the merged data is rendered. The rendering of the data set is based on single channel (e.g. gray-scale intensity) or multi-channel (e.g. Red-Green-Blue (RGB), Hue-Saturation-Value (HSV) and/or Lightness A/B coordinate (LAB)) renderings of the two combined datasets. Combination can be linear or non-linear, additive or multiplicative and can once more involve a combination of various operations such as thresholding, masking and other forms of segmentation. One example is standard multi-channel image representation.

In addition, a user can be provided the ability to change the way the two datasets are merged or displayed (i.e. 3D vs. surface rendering), as well as change or choose which image, or combination of images, gets displayed, using the input device(s) (e.g. mouse, keyboard, joystick, display or other device). Further, the two data sets can be viewed on two different parts of a display or two different displays, if available. The ability to manually match features can be used to initialize the search for an optimal transformation model or to enforce a particular constraint.

It is noted that the data representation is not required to have the same dimensionality as the original dataset. For example, the combined or merged images could be on a surface (2 dimensional) image or enface image, while the original acquired datasets are 3 dimensional.

Several examples of the present disclosure are now provided.

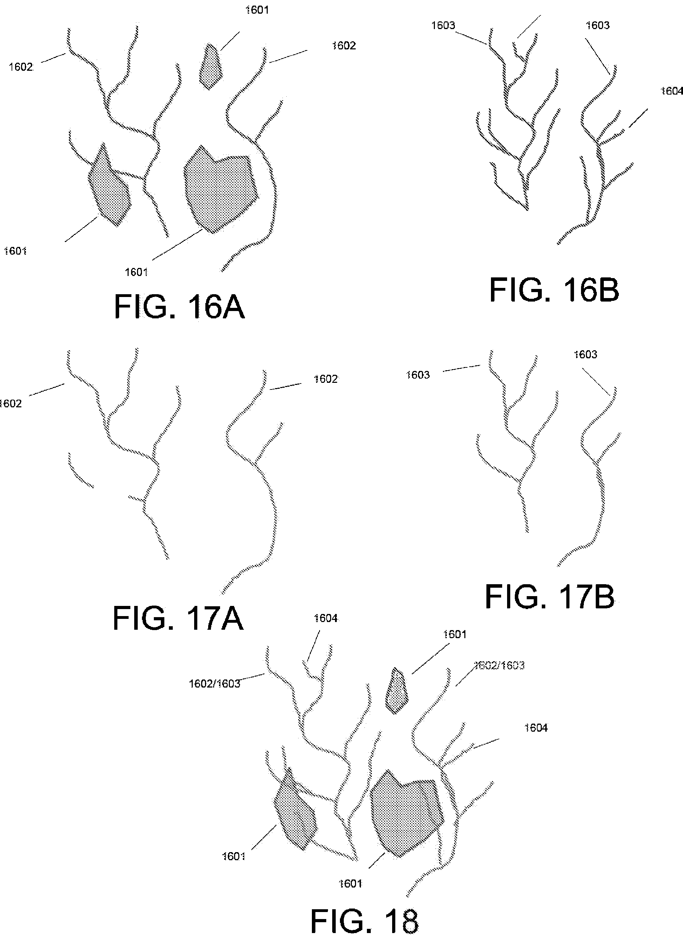

FIGs. 16A, 16B, 17A, 17B and 18 illustrate the process of scaling and/or aligning data sets using features of 2 images. A first probe (i.e. imaging modality 1) produces a first data set (see FIG. 16A) that can produce images having surface patches 1601 and some vasculature structure 1602. A second probe (i.e. imaging modality 2) that produces a second data set (see FIG. 16B) does not produce the surface patches, but produces a more detailed vasculature 1603/1604. The matching features 1602/1603 (i.e. vascular structure common in both images) between two datasets, shown in FIGs. 17A and 17B, is used to merge the two datasets showing all of the features 1601/1602/1603/1604 (see FIG. 18). The merging is achieved by the scaling and/or aligning of the common vascular features using translation, rigid body transformation, or affine transformation, for example, similar to what is described in Lowe, David, International Journal of Computer Vision, Vol. 60 (2004), pp. 91-110.

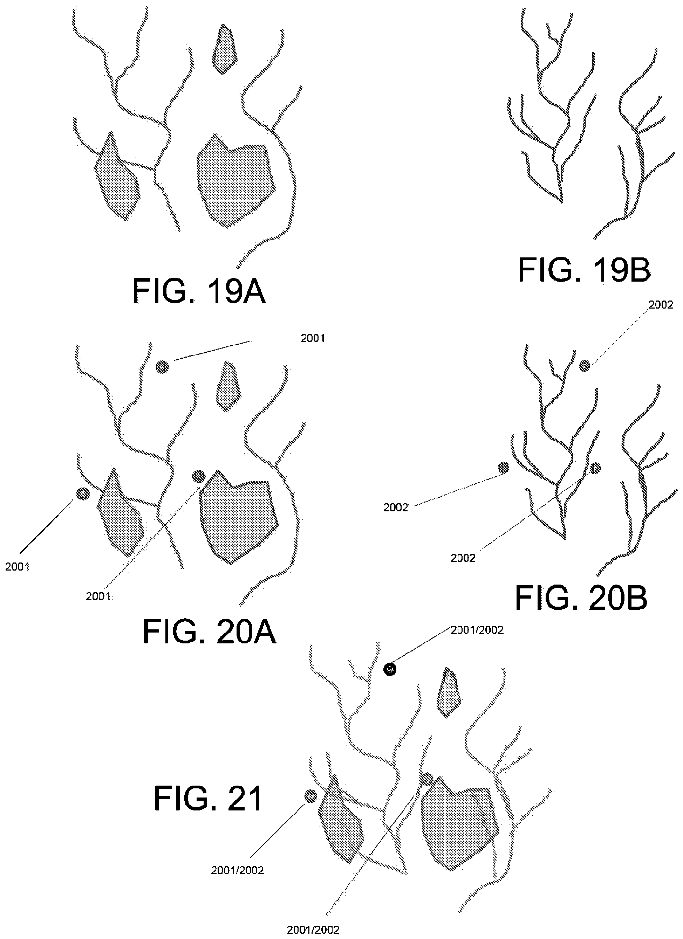

FIGs. 19A, 19B, 20A, 20B and 21 illustrate the process of scaling and/or aligning data sets using features added to 2 images. A first probe (i.e. imaging modality 1) produces a first data set that can produce images having surface patches and some vasculature structure shown in FIG. 19A. A second probe (i.e. imaging modality 2) that produces a second data set does not produce the surface patches, but produces a more detailed vasculature shown in FIG. 19B. Visible features 2001 and 2002 (e.g. a surface mark) are produced in the data sets and are visible in both modalities 1 and 2, as shown in FIGs. 20A and 20B, respectively. The matching features 2001/2002 between two datasets are used to merge the two datasets showing all of the patches and vascular features (see FIG. 21). By scaling and/or aligning the visible features, the images can be merged. Scaling and/or aligning can be performed using translation, rigid body transformation, or affine transformation, for example, similar to what is described in Lowe, David, International Journal of Computer Vision, Vol. 60 (2004), pp. 91-110.

FIGs. 22A, 22B, 23, 24A and 24B illustrate the process of scaling and/or aligning data sets using the same imaging modality but involving images from slightly different locations. A first probe (i.e. imaging modality 1) produces a first data set that can produce images having surface patches and some vasculature structure shown in FIG. 22A. A second probe (i.e. imaging modality 2) that produces a second data set similar, but from a slightly different location, that produces images having surfaces patches and some vasculature structure shown in FIG. 22B. As described above, an interleaving process can be used to produce the final result (see FIG. 23), which is the same as with a single probe scanning all locations, but acquired twice as fast.

As shown in FIGs. 24A and 24B, with a helical pull-back scheme, the two datasets can be combined by assuming each probe is acquiring half of the image at each height. The x axis is the rolled out circumferential axis and the y axis is the longitudinal pull-back direction, e.g. interleaving. That is, computer 130 can utilize a type of interleaving process when combining images from the same types of probes, i.e. the same imaging modes.

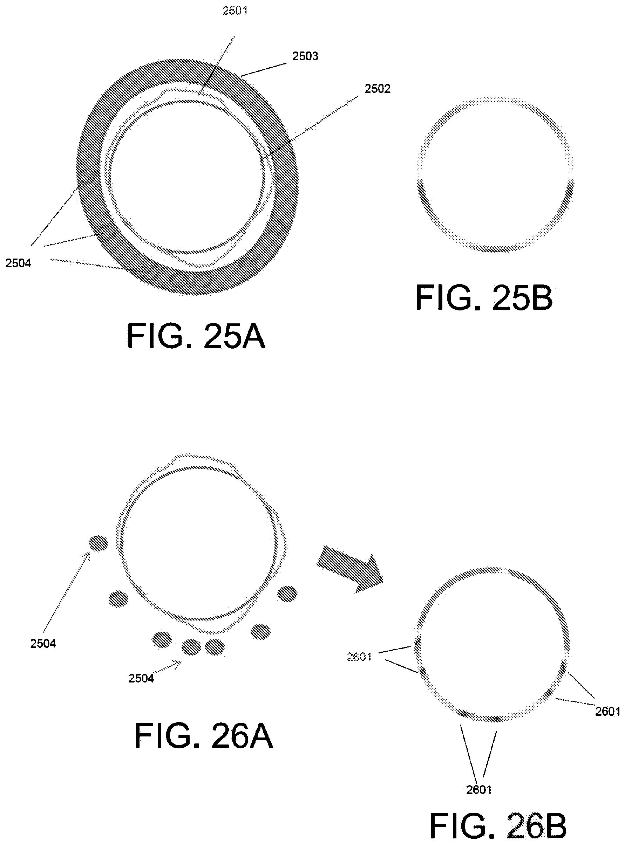

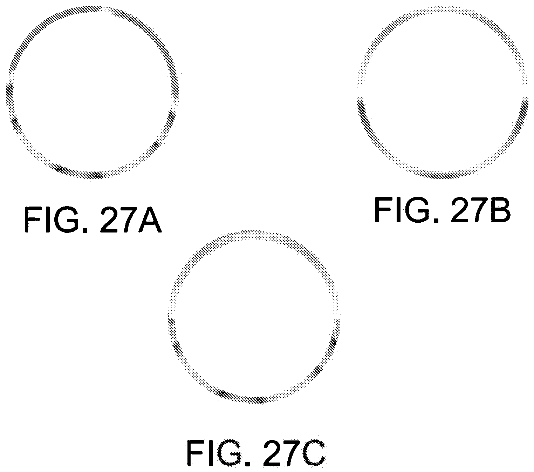

FIGs. 25A, 25B, 26A, 26B, 27A, 27B and 27C illustrate the process of scaling and/or aligning data sets that combines a multiple depth imaging with a single depth image. A full traverse scan at a single height is performed. A first probe (i.e. imaging modality 1) produces a first data set that can produce depth imaging, for example, an OCT scan (see FIG. 25A). This produces an image showing the tissue surface 2501, the balloon 2502 and the sub-surface tissue 2503 and its features 2504. A second probe (i.e. imaging modality 2) that produces a second data set of a tissue surface reflectivity scan, for example, a white light scan (see FIG. 25B). On the depth image data, the processor locates sub-surface features 2504 and pin-points their location on the surface data set (see FIG. 26A). As shown in FIG. 26B, the dark portions 2601 indicate potential features below the surface. FIG. 27A is the sub-surface feature locator of the first probe and FIG. 27B is the surface reflectivity of the second probe. The computer combines the images produced by the two imaging modalities for each slice (see FIG. 27C). For the full sample image (2D surface), a correlation is performed between the surface reflectivity with sub-surface features. In addition, the tissue surface shape can be detected from the depth imaging modality, and use that shape instead of a perfect circle.

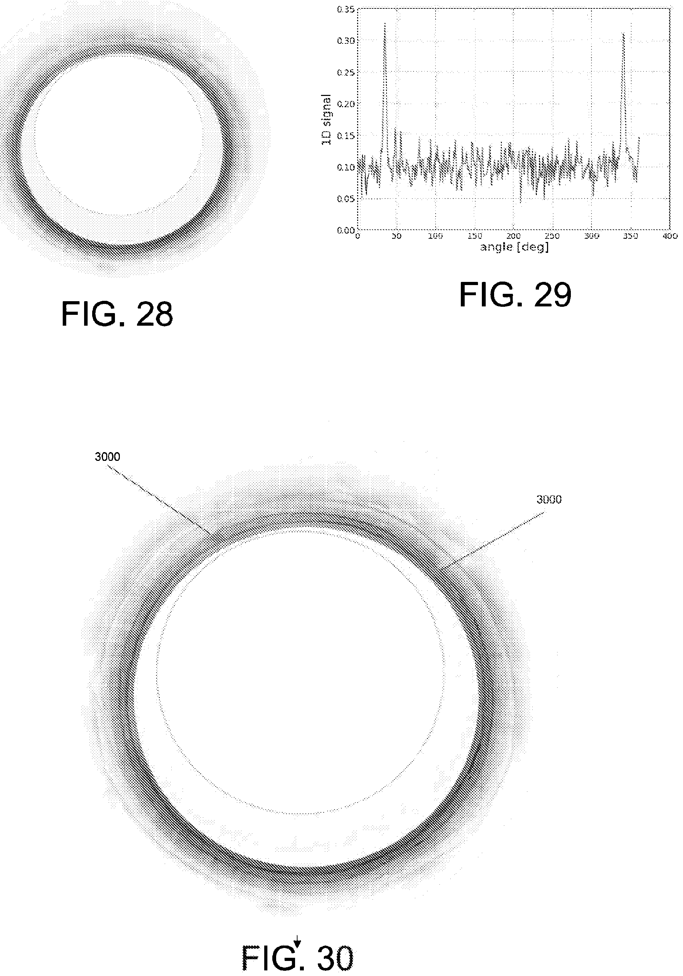

FIGs. 28-30 illustrate an example of such a correlation process. One imaging modality provides full cross-sectional 2D imaging at each longitudinal position by receiving a multiple depth signal at each angular value, using for example Optical Frequency Domain Imaging (OFDI) as the imaging modality. Another imaging modality measures a single return intensity for each angle by using a wavelength that responds strongly to blood vessels just below the surface, using for example Narrow Band Imaging (NBI). As shown in FIG. 28, an OFDI scan produces an image showing tissue layers. Most blood vessels are expected to be in the second layer from the surface, but are a difficult feature to consistently observe. FIG. 29 is a typical return signal from an NBI scan, which shows higher peaks around 40 degrees and 340 degrees. By combining these two images and their respective information, i.e. likely depth location of blood vessels in OFDI and angular location from the NBI signal, FIG. 30 can be produced more precisely locating the blood vessels 3000.

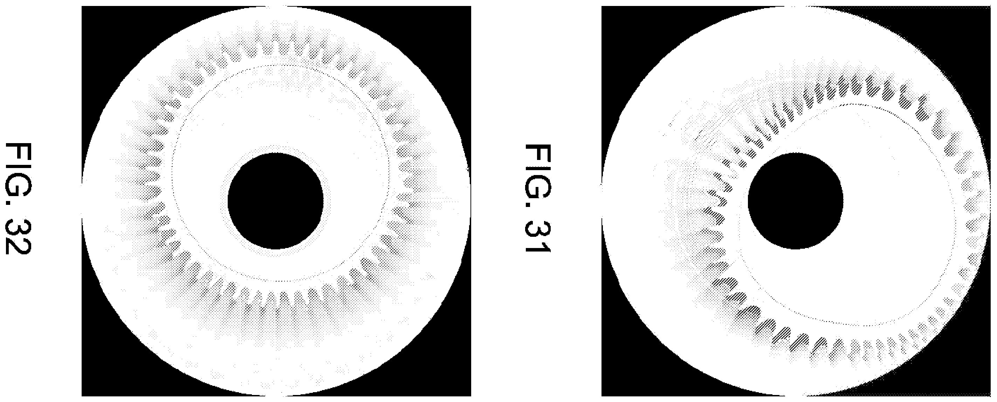

FIGS. 31-32 are diagrams illustrating Non-Uniform Rotational Distortion (NURD) that occurs in images. In one embodiment, one of the probes used can correct for NURD artifacts. In this embodiment, one of the apertures is used to image regularly spaced angular distal encoder, which enables the quantification of the angular position and velocity of the distal end of the imaging optics. One use of this quantification is that the rotating motor is positioned relatively far from the distal optics. This configuration leads to non-uniform rotation at the distal end of the catheter which produces distortion, commonly referred to as Non-Uniform Rotational Distortion (NURD). FIGs. 31-32 are images that represent regularly spaced angular encoders. In FIG. 32, there was very little NURD, while in FIG. 31 there is a lot.

In order to correct for NURD artifacts, the following steps are performed. First, both images are acquired. Next, the angular encoder image is processed to track where each tooth or encoder was located in the image. Then, the non-rigid transformation that would transform the non-uniform encoder image into a regularly-spaced encoder image is computed. Finally, that same transformation is used to transform the actual image of the tissue of interest.

The components of the system can be fabricated from materials suitable for medical applications, including glasses, plastics, polished optics, metals, synthetic polymers and ceramics, and/or their composites, depending on the particular application. For example, the components of the system, individually or collectively, can be fabricated from materials such as polycarbonates such as Lexan 1130, Lexan HPS26, Makrolon 3158, or Makrolon 2458, such as polyether Imides such as Ultem 1010, and/or such as polyethersulfones such as RTP 1400.

Various components of the system may be fabricated from material composites, including the above materials, to achieve various desired characteristics such as strength, rigidity, elasticity, flexibility, compliance, biomechanical performance, durability and radiolucency or imaging preference. The components of the system, individually or collectively, may also be fabricated from a heterogeneous material such as a combination of two or more of the above-described materials. Although embodiments of the present disclosure have been illustrated as separate pieces attached together, the probes can also be constructed as a single element with multiple apertures.

The present disclosure has been described herein in connection with an optical imaging system including an OCT probe. Other applications are contemplated.

Where this application has listed the steps of a method or procedure in a specific order, it may be possible, or even expedient in certain circumstances, to change the order in which some steps are performed, and it is intended that the particular steps of the method or procedure claim set forth herebelow not be construed as being order-specific unless such order specificity is expressly stated in the claim.

The invention is defined by the appended claims.

## Claims

1. A multiple modal optical system, comprising:
an optical subsystem including a probe positioned at a position about a longitudinal axis,
the probe being coupled to one optical fiber (11, 111); and
at least two light sources (19, 119) respectively emitting first light (La) and second light (Lb),
connectable to the optical fiber (11, 111) of the optical subsystem, wherein the first light and the second light travel over the one optical fiber (11, 111) through a single prism (15,115)
wherein the multiple modal optical system is configured to: transmit the first light and the second light from the at least two light sources (19, 119) through the optical subsystem in at least one direction transverse to the longitudinal axis to irradiate an object and receive first reflected light and second reflected light from the object,
wherein the first light (La) and second light (Lb) have parallel optical axes;
wherein the first reflected light and the second reflected light travel back over the optical fiber (11, 111), and the first reflected light and the second reflected light correspond to the first light and the second light, respectively, and wherein the optical subsystem is configured to: rotate about the longitudinal axis; and translate along the longitudinal axis when connected to the at least two light sources (19, 119).

2. The multiple modal optical system of claim 1, further comprising:
a processor configured to:
receive first data representative of the first reflected light;
receive second data representative of the second reflected light,
said first data and said second data being representative of a common tissue sample of the object,
said first data and said second data including a common feature related to the common tissue sample;
identify the common feature in the first data and the second data; and
modify the first data to at least one of register, align or scale an image produced by the first data to an image produced by the second data based on the common feature.

3. The multiple modal optical system of claim 1, wherein a first of the at least two light sources (19, 119) is a coherent light source for an optical coherence tomography imaging modality.

4. The multiple modal optical system of claim 3, wherein a second of the at least two light sources (19, 119) is one of coherent, visible and infrared light source.

5. A system for generating an image using the multiple modal optical system of claim 1, comprising: a first detector, a second detector and a processor, wherein the optical subsystem further comprises another probe connected to the optical fiber (11, 111),
wherein the probe is connectable to a first of the at least two light sources (19, 119), and is configured to: receive the first light from the first light source and exit the first light in a first direction transverse to the longitudinal axis to irradiate a first portion of the object; and receive the first reflected light from the first portion of the object, and
wherein the another probe is positioned about the longitudinal axis at another position at or about the position of the probe, is connectable to a second of the at least two light sources (19, 119), and is configured to: receive the second light from the second light source and exit the second light in a second direction transverse to the longitudinal axis to irradiate a second portion of the object, the second direction being different from the first direction; and receive the second reflected light from the second portion of the object,
wherein the first detector is configured to receive the first reflected light and convert the first detected light into a first signal, and the second detector configured to receive the second reflected light and convert the second detected light into a second signal;
wherein the first reflected light and second reflected light have parallel optical axes; and
wherein the processor is configured to:
receive first data and second data representative of the first signal and the second signal,
respectively, said first data and said second data representative of a common tissue sample, said first signal and said second signal including a common feature related to the common tissue sample,
identify the common feature in the first data and the second data, and
modify the first data to at least one of register, align or scale an image produced by the first data to an image produced by the second data based on the common feature.

6. A method for generating an image using the multiple modal optical system of claim 1, comprising:
receiving, by at least one detector, first data and second data representative of the first reflected light and the second reflected light, respectively, said first data and said second data representative of a common tissue sample, said first data and said second data including a common feature related to the common tissue sample;
identifying, by a processor, the common feature in the first data and the second data; and
modifying, by the processor, the first data to at least one of register, align or scale an image produced by the first data to an image produced by the second data based on the common feature.

7. The multiple modal optical system of claim 1, wherein the multiple modal optical system is configured to transmit the first light and the second light through the optical subsystem in different directions transverse to the longitudinal axis to irradiate different locations of the object.

8. The multiple modal optical system of claim 1, wherein the probe comprises at least one of a grin lens (14), a spacer (16) and a prism (15).

9. The multiple modal optical system of claim 1, wherein the probe corresponds to an optical coherence tomography probe, and the another probe corresponds to a laser marking probe.

10. The multiple modal optical system of claim 1, wherein the object is a tissue, and the laser marking probe is used to mark the tissue for later identification in an image for positional reference or removal of the tissue.

11. The multiple modal optical system of claim 1, wherein the optical subsystem further comprises another probe connected to the optical fiber (11, 111),
wherein the probe is connectable to a first of the at least two light sources, and is configured to: receive the first light from the first light source and exit the first light in a first direction transverse to the longitudinal axis to irradiate a first portion of the object; and receive the first reflected light from the first portion of the object, and
wherein the another probe is positioned about the longitudinal axis at another position at or about the position of the probe, is connectable to a second of the at least two light sources, and is configured to: receive the second light from the second light source and exit the second light in a second direction transverse to the longitudinal axis to irradiate a second portion of the object, the second direction being different from the first direction; and receive the second reflected light from the second portion of the object.

12. The multiple modal optical system of claim 1, further comprising:
a processor configured to:
receive first data representative of the first reflected light;
receive second data representative of the second reflected light, said first data and said second data being representative of a common tissue sample of the object;
identify the common feature in the first data and the second data; and
modify the first data to at least one of register, align or scale an image produced by the first data to an image produced by the second data based on the common feature.

## Patentansprüche

1. Multimodales optisches System, das Folgendes umfasst:
ein optisches Teilsystem mit einer Sonde, die in einer Position um eine Längsachse herum angeordnet ist, wobei die Sonde mit einer optischen Faser (11, 111) gekoppelt ist, und
mindestens zwei Lichtquellen (19, 119), die erstes Licht (La) beziehungsweise zweites Licht (Lb) emittieren und mit der optischen Faser (11, 111) des optischen Teilsystems verbindbar sind, wobei das erste und das zweite Licht über die eine optische Faser (11, 111) ein einzelnes Prisma (15, 115) durchläuft,
wobei das multimodale optische System so konfiguriert ist, dass es: das erste und das zweite Licht aus den mindestens zwei Lichtquellen (19, 119) durch das optische Teilsystem in mindestens einer quer zur Längsachse verlaufenden Richtung aussendet und so ein Objekt bestrahlt und von dem Objekt erstes und zweites reflektiertes Licht empfängt,
wobei das erste Licht (La) und das zweite Licht (Lb) parallele optische Achsen aufweisen,
wobei das erste und das zweite reflektierte Licht über die optische Faser (11, 111) zurückläuft und das erste und das zweite reflektierte Licht dem ersten beziehungsweise dem zweiten Licht entspricht und wobei das optische Teilsystem so konfiguriert ist,
dass es: sich um die Längsachse dreht und sich entlang der Längsachse verschiebt,
wenn es mit den mindestens zwei Lichtquellen (19, 119) verbunden ist.

2. Multimodales optisches System nach Anspruch 1, das ferner Folgendes umfasst:
einen Prozessor, der so konfiguriert ist, dass er:
erste Daten empfängt, die das erste reflektierte Licht repräsentieren,
zweite Daten empfängt, die das zweite reflektierte Licht repräsentieren,
wobei die ersten und die zweiten Daten eine gemeinsame Gewebeprobe von dem Objekt repräsentieren,
wobei die ersten und die zweiten Daten ein gemeinsames Merkmal aufweisen, das die gemeinsame Gewebeprobe betrifft,
das gemeinsame Merkmal in den ersten und den zweiten Daten identifiziert und
die ersten Daten so modifiziert, dass ein mit den ersten Daten erzeugtes Bild auf der Grundlage des gemeinsamen Merkmals auf ein Bild registriert, ausgerichtet oder/und
skaliert wird, das mit den zweiten Daten erzeugt wird.

3. Multimodales optisches System nach Anspruch 1, wobei es sich bei einer ersten der mindestens zwei Lichtquellen (19, 119) um eine kohärente Lichtquelle für eine optische Kohärenztomografiebildgebungsmodalität handelt.

4. Multimodales optisches System nach Anspruch 3, wobei es sich bei einer zweiten der mindestens zwei Lichtquellen (19, 119) um eine Quelle für kohärentes, sichtbares oder Infrarotlicht handelt.

5. System zum Erzeugen eines Bildes unter Verwendung des multimodalen optischen Systems nach Anspruch 1 mit Folgendem: einem ersten Detektor, einem zweiten Detektor und einem Prozessor, wobei das optische Teilsystem ferner eine weitere Sonde umfasst, die mit der optischen Faser (11, 111) verbunden ist,
wobei sich die Sonde mit einer ersten der mindestens zwei Lichtquellen (19, 119) verbinden lässt und so konfiguriert ist, dass sie: das erste Licht aus der ersten Lichtquelle empfängt und in einer quer zur Längsachse verlaufenden ersten Richtung austreten lässt und so einen ersten Abschnitt des Objekts bestrahlt und das erste reflektierte Licht von dem ersten Abschnitt des Objekts empfängt, und
wobei die weitere Sonde in einer anderen Position in der oder um die Position der Sonde um die Längsachse herum angeordnet ist, sich mit einer zweiten der mindestens zwei Lichtquellen (19, 119) verbinden lässt und so konfiguriert ist, dass sie: das zweite Licht aus der zweiten Lichtquelle empfängt und in einer quer zur Längsachse verlaufenden zweiten Richtung austreten lässt und so einen zweiten Abschnitt des Objekts bestrahlt, wobei sich die zweite Richtung von der ersten Richtung unterscheidet, und das zweite reflektierte Licht von dem zweiten Abschnitt des Objekts empfängt,
wobei der erste Detektor so konfiguriert ist, dass er das erste reflektierte Licht empfängt und das erste detektierte Licht in ein erstes Signal umwandelt, und der zweite Detektor so konfiguriert ist, dass er das zweite reflektierte Licht empfängt und das zweite detektierte Licht in ein zweites Signal umwandelt,
wobei das erste und das zweite reflektierte Licht parallele optische Achsen aufweisen und
wobei der Prozessor so konfiguriert ist, dass er:
erste und zweite Daten empfängt, die das erste beziehungsweise das zweite Signal repräsentieren, wobei die ersten und die zweiten Daten eine gemeinsame Gewebeprobe repräsentieren, wobei das erste und das zweite Signal ein gemeinsames Merkmal aufweisen, das die gemeinsame Gewebeprobe betrifft,
das gemeinsame Merkmal in den ersten und den zweiten Daten identifiziert und
die ersten Daten so modifiziert, dass ein mit den ersten Daten erzeugtes Bild auf der Grundlage des gemeinsamen Merkmals auf ein Bild registriert, ausgerichtet oder/und
skaliert wird, das mit den zweiten Daten erzeugt wird.

6. Verfahren zum Erzeugen eines Bildes unter Verwendung des multimodalen optischen Systems nach Anspruch 1, das Folgendes umfasst:
Empfangen erster und zweiter Daten durch mindestens einen Detektor, die das erste beziehungsweise das zweite reflektierte Licht repräsentieren, wobei die ersten und die zweiten Daten eine gemeinsame Gewebeprobe repräsentieren, wobei die ersten und die zweiten Daten ein gemeinsames Merkmal aufweisen, das die gemeinsame Gewebeprobe betrifft,
Identifizieren des gemeinsamen Merkmals in den ersten und den zweiten Daten durch einen Prozessor und
derartiges Modifizieren, durch den Prozessor, der ersten Daten, dass ein mit den ersten Daten erzeugtes Bild auf der Grundlage des gemeinsamen Merkmals auf ein Bild registriert, ausgerichtet oder/und skaliert wird, das mit den zweiten Daten erzeugt wird.

7. Multimodales optisches System nach Anspruch 1, wobei das multimodale optische System so konfiguriert ist, dass es das erste und das zweite Licht durch das optische Teilsystem in unterschiedlichen Richtungen quer zur Längsachse aussendet und so verschiedene Stellen am Objekt bestrahlt.

8. Multimodales optisches System nach Anspruch 1, wobei die Sonde eine GRIN-Linse (14), einen Abstandhalter (16) oder/und ein Prisma (15) umfasst.

9. Multimodales optisches System nach Anspruch 1, wobei die Sonde einer Sonde für optische Kohärenztomografie und die weitere Sonde einer Lasermarkierungssonde entspricht.

10. Multimodales optisches System nach Anspruch 1, wobei es sich bei dem Objekt um ein Gewebe handelt und die Lasermarkierungssonde zum Markieren des Gewebes zur späteren Identifizierung in einem Bild für die Positionsangabe oder das Entfernen des Gewebes benutzt wird.

11. Multimodales optisches System nach Anspruch 1, wobei das optische Teilsystem ferner eine weitere Sonde umfasst, die mit der optischen Faser (11, 111) verbunden ist,
wobei sich die Sonde mit einer ersten der mindestens zwei Lichtquellen verbinden lässt und so konfiguriert ist, dass sie: das erste Licht aus der ersten Lichtquelle empfängt und in einer quer zur Längsachse verlaufenden ersten Richtung austreten lässt und so einen ersten Abschnitt des Objekts bestrahlt und das erste reflektierte Licht von dem ersten Abschnitt des Objekts empfängt, und
wobei die weitere Sonde in einer anderen Position in der oder um die Position der Sonde um die Längsachse herum angeordnet ist, sich mit einer zweiten der mindestens zwei Lichtquellen verbinden lässt und so konfiguriert ist, dass sie: das zweite Licht aus der zweiten Lichtquelle empfängt und in einer quer zur Längsachse verlaufenden zweiten Richtung austreten lässt und so einen zweiten Abschnitt des Objekts bestrahlt, wobei sich die zweite Richtung von der ersten Richtung unterscheidet, und das zweite reflektierte Licht von dem zweiten Abschnitt des Objekts empfängt.

12. Multimodales optisches System nach Anspruch 1, das ferner Folgendes umfasst:
einen Prozessor, der so konfiguriert ist, dass er:
erste Daten empfängt, die das erste reflektierte Licht repräsentieren,
zweite Daten empfängt, die das zweite reflektierte Licht repräsentieren, wobei die ersten und die zweiten Daten eine gemeinsame Gewebeprobe von dem Objekt repräsentieren, das gemeinsame Merkmal in den ersten und den zweiten Daten identifiziert und
die ersten Daten so modifiziert, dass ein mit den ersten Daten erzeugtes Bild auf der Grundlage des gemeinsamen Merkmals auf ein Bild registriert, ausgerichtet oder/und
skaliert wird, das mit den zweiten Daten erzeugt wird.

## Revendications

1. Système optique multimodal, comprenant :
un sous-système optique incluant une sonde positionnée au niveau d'une position autour d'un axe longitudinal, la sonde étant couplée à une fibre optique (11, 111) ; et
au moins deux sources de lumière (19, 119) émettant respectivement une première lumière (La) et une deuxième lumière (Lb), pouvant être connectées à la fibre optique (11, 111) du sous-système optique, la première lumière et la deuxième lumière se déplaçant dans la fibre optique (11, 111) à travers un prisme unique (15, 115),
le système optique multimodal étant conçu pour : émettre la première lumière et la deuxième lumière depuis les au moins deux sources de lumière (19, 119) à travers le sous-système optique dans au moins une direction transversale par rapport à l'axe longitudinal pour éclairer un objet et recevoir une première lumière réfléchie et une deuxième lumière réfléchie de l'objet,
la première lumière (La) et la deuxième lumière (Lb) ayant des axes optiques parallèles ;
la première lumière réfléchie et la deuxième lumière réfléchie revenant par la fibre optique (11, 111), et la première lumière réfléchie et la deuxième lumière réfléchie correspondant à la première lumière et la deuxième lumière, respectivement, et le sous-système optique étant conçu pour : tourner autour de l'axe longitudinal ; et être translaté le long de l'axe longitudinal quand il est connecté aux au moins deux sources de lumière (19, 119).

2. Système optique multimodal selon la revendication 1, comprenant en outre :
un processeur conçu pour :
recevoir des premières données représentatives de la première lumière réfléchie ;
recevoir des deuxièmes données représentatives de la deuxième lumière réfléchie,
lesdites premières données et lesdites deuxièmes données étant représentatives d'un échantillon de tissu commun de l'objet,
lesdites premières données et lesdites deuxièmes données incluant une caractéristique commune se rapportant à l'échantillon de tissu commun ;
identifier la caractéristique commune dans les premières données et les deuxièmes données ; et
modifier les premières données pour au moins l'un d'enregistrer, aligner ou modifier l'échelle d'une image produite par les premières données par rapport à une image produite par les deuxièmes données sur la base de la caractéristique commune.

3. Système optique multimodal selon la revendication 1, dans lequel une première des au moins deux sources de lumière (19, 119) est une source de lumière cohérente destinée à une modalité d'imagerie de tomographie par cohérence optique.

4. Système optique multimodal selon la revendication 3, dans lequel une deuxième des au moins deux sources de lumière (19, 119) est l'une d'une source de lumière cohérente, visible et infrarouge.

5. Système pour la génération d'une image à l'aide du système optique multimodal selon la revendication 1, comprenant : un premier détecteur, un deuxième détecteur et un processeur, le sous-système optique comprenant en outre une autre sonde connectée à la fibre optique (11, 111), la sonde pouvant être connectée à une première des au moins deux sources de lumière (19, 119), et étant conçue pour : recevoir la première lumière de la première source de lumière et émettre la première lumière dans une première direction transversale par rapport à l'axe longitudinal pour éclairer une première partie de l'objet ; et recevoir la première lumière réfléchie de la première partie de l'objet, et
l'autre sonde étant positionnée autour de l'axe longitudinal au niveau d'une autre position au niveau ou autour de la position de la sonde, pouvant être connectée à une deuxième des au moins deux sources de lumière (19, 119), et étant conçue pour : recevoir la deuxième lumière de la deuxième source de lumière et émettre la deuxième lumière dans une deuxième direction transversale par rapport à l'axe longitudinal pour éclairer une deuxième partie de l'objet, la deuxième direction étant différente de la première direction ; et recevoir la deuxième lumière réfléchie de la deuxième partie de l'objet,
le premier détecteur étant conçu pour recevoir la première lumière réfléchie et convertir la première lumière détectée en un premier signal, et le deuxième détecteur étant conçu pour recevoir la deuxième lumière réfléchie et convertir la deuxième lumière détectée en un deuxième signal ;
la première lumière réfléchie et la deuxième lumière réfléchie ayant des axes optiques parallèles ; et le processeur étant conçu pour :
recevoir des premières données et des deuxièmes données représentatives du premier signal et du deuxième signal, respectivement, lesdites premières données et lesdites deuxièmes données étant représentatives d'un échantillon de tissu commun, ledit premier signal et ledit deuxième signal incluant une caractéristique commune se rapportant à l'échantillon de tissu commun,
identifier la caractéristique commune dans les premières données et les deuxièmes données, et
modifier les premières données pour au moins l'un d'enregistrer, aligner ou modifier l'échelle d'une image produite par les premières données par rapport à une image produite par les deuxièmes données sur la base de la caractéristique commune.

6. Procédé de génération d'une image à l'aide du système optique multimodal selon la revendication 1, comprenant les étapes consistant à :
recevoir, à l'aide d'au moins un détecteur, des premières données et des deuxièmes données représentatives de la première lumière réfléchie et de la deuxième lumière réfléchie, respectivement, lesdites premières données et lesdites deuxièmes données étant représentatives d'un échantillon de tissu commun, lesdites premières données et lesdites deuxièmes données incluant une caractéristique commune se rapportant à l'échantillon de tissu commun ;
identifier, à l'aide d'un processeur, la caractéristique commune dans les premières données et les deuxièmes données ; et modifier, à l'aide du processeur, les premières données pour au moins l'un d'enregistrer, aligner ou modifier l'échelle d'une image produite par les premières données par rapport à une image produite par les deuxièmes données sur la base de la caractéristique commune.

7. Système optique multimodal selon la revendication 1, dans lequel le système optique multimodal est conçu pour émettre la première lumière et la deuxième lumière à travers le sous-système optique dans des directions différentes transversales par rapport à l'axe longitudinal pour éclairer différents emplacements de l'objet.

8. Système optique multimodal selon la revendication 1, dans lequel la sonde comprend au moins l'un d'une lentille à gradient d'indice (14), d'un espaceur (16) et d'un prisme (15).

9. Système optique multimodal selon la revendication 1, dans lequel la sonde correspond à une sonde de tomographie par cohérence optique, et l'autre sonde correspond à une sonde de marquage laser.

10. Système optique multimodal selon la revendication 1, dans lequel l'objet est un tissu et la sonde de marquage laser est utilisée pour marquer le tissu pour identification ultérieure dans une image à des fins de référence de position ou d'élimination du tissu.

11. Système optique multimodal selon la revendication 1, dans lequel le sous-système optique comprend en outre une autre sonde connectée à la fibre optique (11, 111),
la sonde pouvant être connectée à une première des au moins deux sources de lumière, et étant conçue pour : recevoir la première lumière de la première source de lumière et émettre la première lumière dans une première direction transversale par rapport à l'axe longitudinal pour éclairer une première partie de l'objet ; et recevoir la première lumière réfléchie de la première partie de l'objet, et
l'autre sonde étant positionnée autour de l'axe longitudinal au niveau d'une autre position au niveau ou autour de la position de la sonde, pouvant être connectée à une deuxième des au moins deux sources de lumière, et étant conçue pour : recevoir la deuxième lumière de la deuxième source de lumière et émettre la deuxième lumière dans une deuxième direction transversale par rapport à l'axe longitudinal pour éclairer une deuxième partie de l'objet, la deuxième direction étant différente de la première direction ; et recevoir la deuxième lumière réfléchie de la deuxième partie de l'objet.

12. Système optique multimodal selon la revendication 1, comprenant en outre :
un processeur conçu pour :
recevoir des premières données représentatives de la première lumière réfléchie ;
recevoir des deuxièmes données représentatives de la deuxième lumière réfléchie, lesdites premières données et lesdites deuxièmes données étant représentatives d'un échantillon de tissu commun de l'objet ;
identifier la caractéristique commune dans les premières données et les deuxièmes données ; et
modifier les premières données pour au moins l'un d'enregistrer, aligner ou modifier l'échelle d'une image produite par les premières données par rapport à une image produite par les deuxièmes données sur la base de la caractéristique commune.
